# EUROPEAN PATENT APPLICATION

(11) **EP 4 491 161 A1**
(43) Date of publication of application: **15.01.2025**
(21) Application number: 23184776.5
(22) Date of filing: 11.07.2023
(51) Int. Cl.: A61F 5/56, A61B 17/04, A61M 25/10, A61B 17/24, A61B 17/06

(54) **AN IMPLANT FOR SUPPORTING SOFT TISSUE**

(71) Applicant: Ostbayerische Technische Hochschule Regensburg, 93049 Regensburg (DE); Universitätsklinikum Regensburg, 93053 Regensburg (DE)
(72) Inventor: KÜHNEL, Thomas, 93053 Regensburg (DE); BURGER, Moritz, 93053 Regensburg (DE); SCHRATZENSTALLER, Thomas, 93053 Regensburg (DE); BARTSCH, Alexander, 93053 Regensburg (DE)
(74) Representative: Lucke, Andreas

(57) **Abstract**

An implant for supporting soft tissue, the implant comprising a distal anchor for implantation in the soft tissue, the distal anchor having a collapsed state and a deployed state, the deployed state having a larger lateral extension than the collapsed state; a connection element attached to the distal anchor for supporting the soft tissue according to a biasing force applied along the connection element; and an expandable element, wherein the expandable element is configured to receive a fluid to expand the expandable element to an expanded deployment state for transferring the distal anchor from the collapsed state to the deployed state; wherein the distal anchor is configured to maintain the deployed state against a restoring force of the tissue of the patient when the expandable element is collapsed from the expanded deployment state

## Description

### FIELD OF THE INVENTION

The present invention is in the field of medical implants for supporting tissue, particularly for biasing tissue towards a bone anchor. More precisely, the present invention relates to implants for the treatment of obstructive sleep apnea.

### BACKGROUND

Obstructive sleep apnea (OSA) is the most common sleep-related breathing disorder and is characterized by recurrent episodes of complete or partial obstruction of the upper airway leading to reduced or absent breathing during sleep. Generally, a reduction of muscle tone in a patient's soft tissue during sleep may induce a partial or complete collapse of a patient's airway, e.g. when a patient's tongue collapses on the back of the throat while the patient is lying on his back.

The collapse of the patient's airway may impede ventilation and reduce oxygen intake of the patient. If reductions in ventilation are associated with sufficiently low blood-oxygen levels or with sufficiently high breathing efforts against an obstructed airway, neurological mechanisms may trigger a sudden interruption of sleep, called a neurological arousal. This arousal can cause an individual to gasp for air and awaken. If the condition persists for extended period of times, a patient's cognitive functions may be severely impacted, while the patient may become conditioned to daytime sleepiness, as well as headaches and fatigue associated with significant levels of sleep disturbance.

Treatment may address the risk factors for obstructive sleep apnea, e.g. through weight reduction of the person and preventing a supine sleep position, or may include therapeutic interventions, such as positive airway pressure, whereby a breathing machine pumps a controlled stream of air through a mask worn over the nose, mouth, or both. However, these methods generally rely on a patient's adherence to the treatment and often require a behavioral change in sleeping habits. Other treatments include neurostimulation, medication, oral appliances, or surgical interventions.

US20080035160A1 teaches a medical implant for implantation in a tongue of a patient, wherein the implant comprises a distal anchor, a tether and a securing assembly, such as bone anchors or screws for attachment to the lower jaw of a patient. The distal anchor is inserted into the tongue close to the patient's airway, and the tether links the distal anchor and the securing assembly for creating tension and preventing a collapse of a patient's airway. The distal anchor is deployed by engaging or piercing the surrounding tissue with hook elements, which are expanded with a pull cord mechanism.

### SUMMARY OF THE INVENTION

The known implants however often rip or puncture the surrounding tissue as part of anchoring the implant in the soft tissue, and the anchors are generally pulled back from the distal end of a surgically created cavity in the tissue during deployment. As a result, severe surgical trauma can prevent rapid recovery of the patient, as the soft tissue, in particular in the tongue of the patient may swell and prevent ventilation. Moreover, the deployment position of the known implants farther away from the throat may result in a shorter tether, which may reduce a patient's ability to freely move his/her tongue or may negatively impact the treatment response. These potential complications also increase the required skill level for a surgeon and can therefore limit the application of the known implants.

In view of this state-of-the-art, the object of the invention is to provide an improved implant for anchoring tissue which can minimize surgical trauma, facilitate the implantation process, and/or improve the control over the deployment configuration of the implant.

This object is solved by an implant according to independent claim 1. The dependent claims relate to preferred embodiments.

According to a first aspect, the invention relates to an implant for supporting soft tissue. The implant comprises a distal anchor for implantation in the soft tissue, a connection element attached to the distal anchor for supporting the soft tissue according to a biasing force applied along the connection element, and an expandable element. The distal anchor has a collapsed state and a deployed state, wherein the deployed state has a larger lateral extension than the collapsed state. The expandable element is configured to receive a fluid to expand the expandable element to an expanded deployment state for transferring the distal anchor from the collapsed state to the deployed state. The distal anchor is configured to maintain the deployed state against a restoring force of the tissue of the patient when the expandable element is collapsed from the expanded deployment state.

The implant can be inserted into the soft tissue with the distal anchor resting on the expandable element, and the fluid, such as a pressurized gas, liquid or gel, may be inserted into the expandable element to transfer the expandable element to the expanded deployment state for transferring the distal anchor to the deployed state. For example, the expandable element may expand from an initial collapsed state to a greater volume in the expanded deployment state to deflect the soft tissue surrounding the expandable element and to deflect sections of the distal anchor on an outer surface of the expandable element. In other words, the outer surface of the expandable element may expand the distal anchor from the collapsed state to the deployed state against a restoring force of the tissue.

The increased lateral extension of the distal anchor in the deployed state may correspond to a larger maximum distance of ends of the distal anchor in the radial direction of the distal anchor, wherein the radial direction may extend radially with respect to an axis along the biasing force. As a result, the deployed state may have a larger radial extension when viewed along an implantation direction and/or an extension direction of the connection element, along which the biasing force is applied to support the soft tissue. The distal anchor may comprise a plurality of bars, which may radially protrude away from a center of the distal anchor in the deployed state, and which may pivot away from each other about a central pivot, when the distal anchor is transferred from the collapsed state to the deployed state. A greater extension of the distal anchor in the radial direction may improve an anchoring of the distal anchor, when the expandable element is collapsed.

In some embodiments, bars of the distal anchor may be connected by intermediate linking elements, such as to form a snow-flake or an umbrella-like shape of the distal anchor in the deployed state.

In some embodiments, the distal anchor comprises flaps, which may be unfolded as part of transforming the distal anchor from the collapsed state to the deployed state, and which may result in an umbrella-like shape of the distal anchor, when it is in the deployed state. For example, the distal anchor may comprise a continuous metal sheet portion, wherein the metal sheet portion may comprise segments of varied material properties, such as segments of reduced material thickness, wherein the metal sheet portion may be configured to fold along the segments of varied material properties to transform the distal anchor from the collapsed state towards the deployed state.

The distal anchor may have a substantially elliptic cross-section and may preferably be radially symmetric, such that the deployed state may have a larger diameter than the collapsed state. An increased cross-section of the distal anchor in the deployed state may improve an anchoring of the distal anchor in the soft tissue, whereas a reduced cross-section in the collapsed state may facilitate the introduction of the distal anchor together with the expandable element through a surgical cavity towards the implantation position/site.

When the expandable element is expanded by receiving the fluid, a diameter/perimeter of the expandable element in the expanded deployment state may correspond substantially to a diameter/perimeter of the distal anchor in the deployed state.

The expandable element may expand uniformly when receiving the fluid, such as to uniformly deflect the surrounding tissue, preferably without or with minimal cutting and/or rupturing the soft tissue, e.g. mainly limited to a rupturing in a direction perpendicular to the muscle fiber direction. For example, an initial lumen created by a cannula inserted into the soft tissue, e.g. with a diameter of about 5 mm, may be locally expanded at the expandable element to create a substantially circular cavity with an increased diameter, e.g. with a diameter of about 10 mm, such as between about 7 mm and about 15 mm, when the expandable element is expanded to the expanded deployment state. When the surrounding tissue collapses subsequent to a collapse of the expandable element, the surrounding tissue may contract around the distal anchor, while the distal anchor maintains the deployed state against the restoring forces of the contracting tissue, i.e. the distal anchor does not return to the collapsed state. The skilled person will appreciate that the distal anchor may nonetheless be compressed as a result of the restoring force of the tissue. Thus, a radial extension of the distal anchor in a maximally expanded state may not be maintained in the deployed state, but the distal anchor may be partially compressed, when the expandable element is collapsed, while a radial extension of the distal anchor in the deployed state can remain larger than the radial extension of the distal anchor in the collapsed state. As a result, the distal anchor may be integrated into the soft tissue with minor injury to the soft tissue, such as to minimize post-operative trauma, and may be anchored in the tissue of the patient in the deployed state.

Moreover, the deployment of the distal anchor using an expandable element may prevent partially retracting the distal anchor during deployment, such that the distal anchor may be deployed closer to a distal end of a surgical cavity in the soft tissue. For an implant for treatment of OSA, the distal anchor may be configured to be implanted in the tongue of a patient and may be anchored close to a surface of the tongue opposite the airway of a patient. As a result, a supporting force of the implant may be more reliably transferred to an intended tissue section and/or a depth of an incision in the tissue for introducing the implant into the soft tissue may be minimized.

In the prior art, it was generally preferred to provide the anchor with barbed hooks and/or to rapidly expand implants for puncturing the surrounding tissue and anchoring the distal anchor. However, the inventors found in their experiments with a distal anchor having a substantially snow-flake shaped cross section, which was anchored in muscle tissue of a pig tongue, that the distal anchor could support an anchoring force equal to several times the weight of the tongue while remaining anchored in the muscle tissue, despite being deployed using an expandable element, which was expanded at a comparatively slow deployment speed, and despite lacking barbed hooks. Moreover, expansion of a balloon catheter was found to cause only minimal tissue damage in the muscle tissue of a pig tongue, such that reduced post-operative trauma is expected using the implant according to the first aspect, when compared to prior art solutions involving the rupturing of soft tissue with rapidly deployed anchors. These advantages are expected to reduce pain of the patient, increase healing rate of the tissue and minimize hospital stay, with associated benefits to the patient and the society.

In preferred embodiments, the expandable element comprises an expandable balloon of a balloon catheter.

The balloon may comprise an elastic material, which may uniformly expand in response to an increased internal pressure arising from the influx of the fluid, such as to uniformly deflect the surrounding soft tissue. The distal anchor may be arranged on the balloon in the collapsed state, and expansion of the balloon to the expanded deployment state may transfer the distal anchor to the deployed state. Specifically, an outer surface of the balloon may press against an inner surface of the distal anchor in the collapsed state, such that the distal anchor is expanded towards the deployed state. Preferably, the balloon is a non-compliant balloon, such that an expansion of the balloon may be reliably reproduced independently of the local elasticity of the soft tissue. A non-compliant balloon may expand uniformly over its longitudinal axis and generally may not be expanded past a predetermined maximum diameter. Thus, in some embodiments, the balloon may be configured to expand towards a predetermined maximum diameter in response to the increased internal pressure, and a further expansion of the balloon is prevented, wherein an increase of a pressure of the balloon has a reduced influence on the diameter of the balloon for an expansion of the balloon past the predetermined maximum diameter, when compared to an expansion from the collapsed state towards the predetermined maximum diameter.

In preferred embodiments, the distal anchor is configured to rest on the balloon during implantation of the implant.

The distal anchor may be arranged on the balloon and may be attached to the connection element to be inserted into a cavity in the soft tissue, e.g. through a cannula of a trocar. The combination of the distal anchor and the balloon catheter may be inserted through the cannula towards an implantation position/site and the distal anchor may be subsequently deployed by expanding the balloon of the balloon catheter and applying a pulling force on the connection element, such as to maintain a contact of an outer surface of the balloon and the distal anchor, while the distal anchor is transferred from the collapsed state to the deployed state.

In preferred embodiments, the balloon catheter comprises a guiding slot for guiding the connection element from a distal portion of the balloon catheter to a proximal portion of the balloon catheter.

The guiding slot may secure and/or guide the connection element along an extension of the balloon catheter during implantation, and may guide a pulling force on the connection element to the distal anchor, such as to maintain a contact between the distal anchor and the balloon during a deployment of the distal anchor. The guiding slot may be a channel, which may surround the connection element along the extension of the balloon catheter, and which may substantially extend in parallel to a channel of the balloon catheter for guiding the fluid from a proximal portion of the balloon catheter to the expandable element arranged at a distal portion of the balloon catheter.

In preferred embodiments, the guiding slot is configured to guide a holding force applied to the connection element from the proximal portion of the balloon catheter to the distal anchor, such that the connection element can maintain a contact between the distal anchor and the expandable element, while the expandable element is expanded.

The holding force may maintain a contact of the distal anchor and the balloon, when the expandable element is expanded, to enable an inelastic deformation of a portion of the distal anchor, such that the distal anchor is transferred from the collapsed state to the deployed state. When the expandable element is collapsed, the balloon catheter may be retracted from the cavity in the soft tissue, while the connection element may be unsheathed from the guiding slot.

In preferred embodiments, the distal anchor is crimped onto the expandable element in the collapsed state of the distal anchor prior to deploying the implant.

The distal anchor may comprise flukes and/or flaps which may be crimped onto the balloon of a balloon catheter, such as to surround the balloon, and the flukes and/or flaps may be deflected from a center section of the distal anchor, when the balloon is expanded.

In some embodiments, the distal anchor is configured to be inelastically deformed when transferring the distal anchor from the collapsed state to the deployed state.

The flukes and/or flaps of the distal anchor may expand in an umbrella-like manner, whereby the deflected portions may be inelastically deformed to transfer the distal anchor towards the deployed state. For example, the distal anchor may comprise hinge sections with a reduced material thickness, and the hinge sections may be inelastically deformed to unfold the distal anchor from the collapsed state to the deployed state.

In some embodiments, the expandable element is configured to plastically deform the distal anchor, when the expandable element is expanded.

The expandable element may support a pressure sufficient to plastically deform the distal anchor, which may be larger than a force required to expand a cavity in the soft tissue against the restoring force of muscular tissue. For example, the expandable element may be configured to support a pressure of more than 6 bar, such as 10 bar, to transfer the distal anchor from the collapsed state to the deployed state.

In preferred embodiments, the distal anchor comprises a plurality of folding portions, wherein the distal anchor is configured to fold along the folding portions to transfer the distal anchor from the collapsed state to the deployed state.

The distal anchor should be configured to hold the weight of the tongue both dynamically and statically. The skilled person will appreciate that several options may be used to configure the anchor, which can fulfill said requirement. For example, the distal anchor may comprise a plurality of bars and folding sections, which may form a repeating pattern, in analogy to the design of a coronary stent. Additionally or alternatively, the distal anchor may have an umbrella-like structure, wherein the distal anchor may comprise a structure made up of folded segments, which radially expand around a pivot to assume the deployed configuration. In the latter case, the segments may be discontinuous, e.g. to assume a snowflake-like shape, when the distal anchor is in the deployed state, or continuous, such as to assume an umbrella-like anchor shape.

The distal anchor may be formed of a biocompatible material, and may in particular comprise a biocompatible metal, such as 316L steel or grade 2 titanium.

The folding portions may be pre-defined, such as to define a pre-defined unfolding/collapsing geometry of the distal anchor. The folding portions may be implemented by a laser manufacturing process. For example, local heat treatment of the metal structure may decouple the deformation sequence from the anchor geometry. In some examples, the distal anchor comprises a laser processed material section with locally changed bending properties. Additionally or alternatively, a metal structure of the distal anchor may comprise material portions with reduced material thickness, such as to implemented pre-defined folding portions. The material thickness of a metal structure may be locally changed by local laser processing, such as material ablation via deep metal laser engraving.

As a result, a three-dimensional part may be formed, which may be deformed based on locally varying material properties in response to a uniformly applied pressure arising from the expansion of a balloon catheter, such as to enable a controlled transfer of the distal anchor to the deployed state during implantation, and collapse of the distal anchor under a restoring force of the surrounding tissue may be prevented, when the expandable element is collapsed.

When the distal anchor is implanted in the soft tissue, the connection element can transfer a pulling force to the soft issue through the distal anchor, such as to support the soft tissue along an extension direction of the connection element. The connection element may be attached to a bone portion of a patient, e.g. the lower jaw bone of a patient, by means of a fixation, such as a bone screw, for supporting the soft tissue. The connection element can bias the soft tissue connected to the distal anchor towards the fixation, and may be flexible for allowing natural tissue movement, such as natural tongue movement during speech.

In preferred embodiments, the implant further comprises a switchable element, wherein the switchable element is configured to be switched from a first state to a second state, wherein the first state is associated with a first length, wherein the second state is associated with a second length, and wherein the first length is different from, in particular smaller than, the second length.

The switchable element may be arranged along the connection element, such as to selectively increase a mobility of the soft tissue in which the distal anchor is anchored. Additionally or alternatively, the first state and the second state may be associated with a different elastic modulus, e.g. the Young's modulus of the switchable element arranged along the connection element. As a result, the implant may be switched in an implanted state from the first state to the second state to selectively increase a mobility of the soft tissue supported by the implant.

In preferred embodiments, the switchable element comprises a switch and actuating the switch transforms the switchable element from the first state to the second state.

For example, the switchable element may comprise a push element, which may be actuated by a patient by pressing on the skin overlying the implant, such as to selectively increase or decrease an effective length of the connection element, e.g. to increase tissue mobility of the soft tissue. For example, a patient may actuate a switch by pressing on a skin portion close to a lower jaw section of the patient to increase a length of the connection element connecting the distal anchor implanted in the tongue of the patient to the fixation attached to the lower jaw bone.

In preferred embodiments, the switch is configured to urge a latch mechanism to switch back and forth between two configurations, when the switch is actuated, such that the switchable element switches from the first state to the second state, and vice-versa.

For example, the switch may be based on a retractable pen mechanism, wherein actuation of the switch against a spring force may switch between a "retracted position" and an "extended position" of the latch mechanism. The skilled person will appreciate that corresponding latch mechanisms do not require an actuation of the switch in the direction of the length change, as in the case of a standard retractable pen, but the switch may be actuated along a direction perpendicular to an extension direction of the connection element, just to give an example. One or both of the two configurations may be metastable, such that an actuation force on the switch initiates a state transfer between the two configurations.

In addition, the switchable element may be actuated to switch between the first state and the second state based on a physiological movement of the patient, such as a characteristic muscle movement by the patient. For example, for a tongue implant, the patient may perform a characteristic tongue movement, such as to roll up or stick out his/her tongue for actuating a mechanical switch of the switching element. For a tongue implant, the force threshold for the switch may be configured such that inadvertent actuation during sleep, in which muscle tone is generally lower, is prevented, but such that a patient may deterministically actuate the switch based on a conscious muscle contraction and/or selective actuation.

In preferred embodiments, the switchable element is configured to be switched from the first state to the second state based on a muscle contraction of the patient.

In some embodiments, the muscle contraction may switch the switchable element back and forth between the first state and the second state. For example, a tongue movement may compress a portion of the switchable element for actuating a switch, which may mediate a transfer from the first state to the second state, and vice-versa.

In some embodiments, the switchable element comprises a switch for transferring the switchable element from the first state to the second state and a second return mechanism for transferring the switchable element from the second state to the first state, wherein the switch and the return mechanism are the same or different.

In preferred embodiments, the switchable element comprises a return mechanism, wherein the return mechanism returns the switchable element from the second state to the first state after a predetermined time period.

The predetermined time-period may be defined by a mechanical element associated with the switching element, such as a viscoelastic mechanism, which may induce a change of the state of the switching element after a pre-determined elapsed time, wherein the elapsed time may be based on a maximum flow rate of an element of the viscoelastic mechanism.

In preferred embodiments, the return mechanism comprises a viscoelastic element, wherein a gel or liquid flows from an expander vessel to a storage vessel to return the switchable element from the second state to the first state after the predetermined time period.

The flow from the expander vessel to the storage vessel of the gel or liquid may be limited by a flow constriction along a fluid connection between the vessels, such that the gel or liquid may only flow at a pre-determined flow rate. The gel or liquid may be a viscous gel or liquid, such as a polymer, and may further be a rheopectic and/or dilatant gel or liquid whose viscosity is dependent on time and/or a rate of shear strain. As an example, the gel or liquid may comprise a boron based silicone, such as a boron-organo-silicon oxide polymer with or without magnetoactive particles. A mechanism comprising a rheopectic liquid as the fluid may be resistant to rapid tissue movement, such as muscle contractions during speech or swallowing for a tongue implant.

The switchable element may comprise a one-way fluid valve arranged along a second fluid connection between the expander vessel and the storage vessel, and a flow of the fluid from the storage vessel to the expander vessel may be initiated by applying pressure to the storage vessel and/or based on a pulling force along the connection element.

The switchable element may comprise a metallic body with a variable length, such as a metal body with predetermined folding portions, wherein portions of the metallic body may snap back and forth between two configurations when the switchable element is transferred between the first state and the second state to adjust an effective length of the connection element.

In preferred embodiments, the switchable element comprises a switching element made of a superelastic alloy, in particular a nickel titanium alloy, and the switching element reversibly deforms to a stressed state, e.g. in which portions of the switching element are in a stress induced phase, to transfer the switchable element from the first state to the second state.

A nickel titanium alloy, such as nitinol, may be biocompatible and may be superelastic, such that the switchable element may reliably switch back and forth between the first state and the second state over a comparatively large number of cycles, e.g. when implanted in a tongue of a patient.

In preferred embodiments, the implant is for treating obstructive sleep apnea, and the distal anchor is configured for implantation in a tongue of a patient.

The distal anchor may be implanted close to the root of the tongue, and preferably close to a surface of the tongue facing the throat of the patient in a mobile section of the tongue, such that a biasing force applied through the connection element may prevent a collapse of the tongue on the patient's airway during sleep. Thus, the implant may prevent the collapse of a patient's airway through an anchoring of tongue tissue.

In preferred embodiments, the connection element comprises an elastic portion, wherein a biasing force of the elastic portion is configured or configurable for allowing tongue movement of the patient, but preventing a gravitational collapse of the patient's airway.

The elastic portion may be implemented as part of the connection element, e.g. by forming portions of the connection element of an elastic material, such as silicone, or may be implemented as an elastic element between substantially inelastic sections of the connection element. For example, the connection element may be formed of or may comprise a section made of braided metal wire embedded in a silicone mantle. The elastic element may apply a biasing force of about 1 N on the tongue for preventing a collapse of the patient's airway during sleep, when the implant is implanted and the connection element is fixed to a bone portion of the patient.

According to a second aspect, the invention relates to an implant kit comprising the implant according to the first aspect, wherein the implant kit further comprises a cannula for introducing the distal anchor on the expandable element into the tongue of a patient.

The cannula may be part of a trocar for implanting the implant, and may comprise a distal tip for puncturing tissue of a patient. The cannula may guide the distal anchor on the expandable element towards a desired implantation location in the tongue of the patient, such that the distal anchor may be transferred from the collapsed state to the deployed state in the tongue of the patient in a minimally invasive manner.

In preferred embodiments, the implant kit further comprises a fixation for fixedly attaching the implant to a bone portion of the patient, in particular to a lower jaw bone portion of the patient, wherein, in a deployed state of the implant, the fixation is connected to the distal anchor through the connection element.

The fixation may be a bone screw made of PEEK or a titanium alloy, or any other suitable biocompatible material. The fixation may support a biasing force selected by a surgeon during implantation, which may bias the distal anchor towards the fixation along the extension of the connection element. A connection between the fixation and the connection element may be implemented by means of a connector, which may be continuously or stepwise adjustable in length, or may be made directly, e.g. by winding the connection element around a portion of the fixation and/or through knots.

In preferred embodiments, the implant kit further comprises a fixation plate configured for attachment of the connection element to a bone portion of the patient.

The fixation plate may provide an attachment section for attaching the connection element to the fixation in a location distanced away from the jaw bone. For example, the attachment section may be configured to be arranged between the jaw bone and the distal anchor.

In preferred embodiments, the fixation plate is configured to protrude towards the tongue of the patient, when the implant is deployed in the tongue of a patient, such that the connection element is prevented from directly contacting a bone portion of the patient, when the distal anchor is biased towards an attachment portion of the fixation plate by the connection element.

The fixation plate may define a point of attachment between the fixation and the connection element and may move a pivot for an end of the connection element towards the tongue, as to reduce friction between the connection element and the bone and/or tissue portions around the bone.

In preferred embodiments, the lateral extension of the distal anchor in the collapsed state and/or the inner diameter and/or the outer diameter of the cannula is smaller than 6 mm.

According to a third aspect, the invention relates to another implant for supporting tissue. The implant comprises a distal anchor for implantation in the soft tissue, a connection element attached to the distal anchor for supporting the soft tissue according to a biasing force applied along the connection element, and a switchable element. The switchable element is connected or connectable to the connection element and can be switched back and forth from a first state to a second state in an implanted configuration of the implant. The first state has a lower length and/or a higher elastic modulus than the second state, such that a mobility of the distal anchor implanted in the soft tissue and biased by the connection element may be selectively increased by switching the switchable element from the first state to the second state.

The implant may comprise any combination of the features of the implant according to the first aspect and any embodiments thereof, such as the features of the distal anchor, and may also be deployed using an expandable element. Further, the implant according to the third aspect may also be used in the implant kit of the second aspect, and a corresponding implant kit may comprise any combination of the features of the embodiments of the second aspect.

### DETAILED DESCRIPTION OF EMBODIMENTS

The features and numerous advantages of the implant and implant kit according to the present invention will best be understood from a detailed description of preferred embodiments with reference to the accompanying drawings, in which:
- Fig. 1: schematically illustrates an example of an implant in a sectional side view of a patient's lower jaw;
- Fig. 2A-3A: illustrate example steps of an implantation process of the implant shown in Fig. 1 in the tongue of a patient based on a schematic sectional side view of a lower jaw;
- Fig. 3B: schematically illustrates an explantation process of the distal anchor with an explantation element according to an example;
- Fig. 4: schematically illustrates a sectional side view of the patient's lower jaw with another example of an implant in an implanted state;
- Fig. 5: schematically illustrates a sectional side view of the patients lower jaw with yet another example of an implant in an implanted state;
- Fig. 6: illustrates a schematic example of the switching of a switchable element between a first state and a second state; and
- Fig. 7: schematically illustrates an example of a return mechanism for a switchable element.

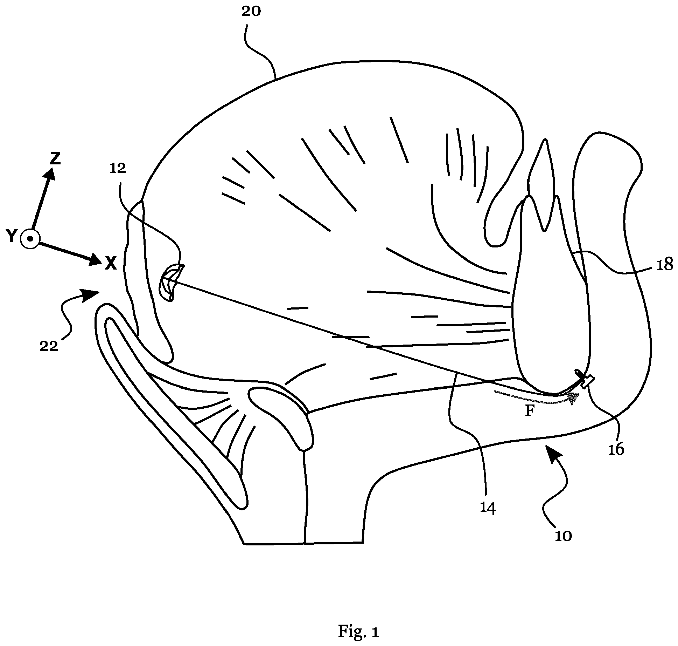

Fig. 1 schematically illustrates a sectional side view of the patient's lower jaw wherein an example of an implant 10 is shown in an implanted state. The implant 10 comprises a distal anchor 12, a connection element 14, and a fixation 16. The fixation 16 is implemented as a bone screw and is fixed to an anterior face of the lower jaw bone 18 of the patient. The distal anchor 12 comprises an umbrella-shaped anchoring element implanted in the tongue 20 of the patient. The distal anchor 12 is arranged close to a back surface 22 of the tongue 20 facing the throat of the patient. The distal anchor 12 is connected to the fixation 16 via the connection element 14, wherein the connection element 14 extends through the soft tissue of the tongue 20 towards the jaw bone 18.

The connection element 14 extends substantially along a biasing direction (indicated as "x" in the drawing), which may be substantially aligned along a direct connection between the fixation 16 and the distal anchor 12. The connection element 14 may impart a biasing force on the distal anchor 12, which may bias the soft tissue of the tongue 20, in which the distal anchor 12 is anchored towards the fixation 16, such that the implant 10 may impede a collapse of the tongue 20 on the patient's airway. At the same time, the connection element 14 may permit movement of the soft tissue, in which the distal anchor 12 is anchored, towards the jaw bone 18 as well as along a direction perpendicular to the biasing direction (x) defined by the extension direction of the connection element 14, e.g. along the directions "y" or "z" illustrated in the drawing.

In the illustrated example, the connection element 14 is implemented as a flexible string-shaped connection, which may be formed of or may comprise an elastic material, such as a flexible connection element 14 comprising a section of braided metal wire embedded in a silicone mantle. Additionally or alternatively, the connection element 14 may comprise an elastic element arranged along the extension direction of the connection element 14, while other sections of the connection element 14 may be effectively inelastic along the extension direction, such as a section of inelastic string.

As a result, the connection element 14 may permit movement along the extension direction of the connection element 14 against a restoring force of the connection element 14. Preferably, an effective Young's modulus of the connection element 14 is selected to permit natural tongue movement involved in swallowing and speech, but also prevents the collapse of the tongue 20 on the patient's airway, e.g. by applying a biasing force F of about 1 N along the extension of the connection element 14 before the back surface 22 of the tongue 20 touches the opposite surface of the throat. A biasing force of about 1 N may counteract a weight of the tongue 20 in the supine position of the patient. In some embodiments, the connection element 14 is preloaded with a biasing force of between about 0.1 N and 1 N during implantation.

The implant 10 may be implanted in a minimally invasive manner through the lower jaw of the patient, and may be arranged completely inside of the tissue of the patient for minimizing post-operative complications.

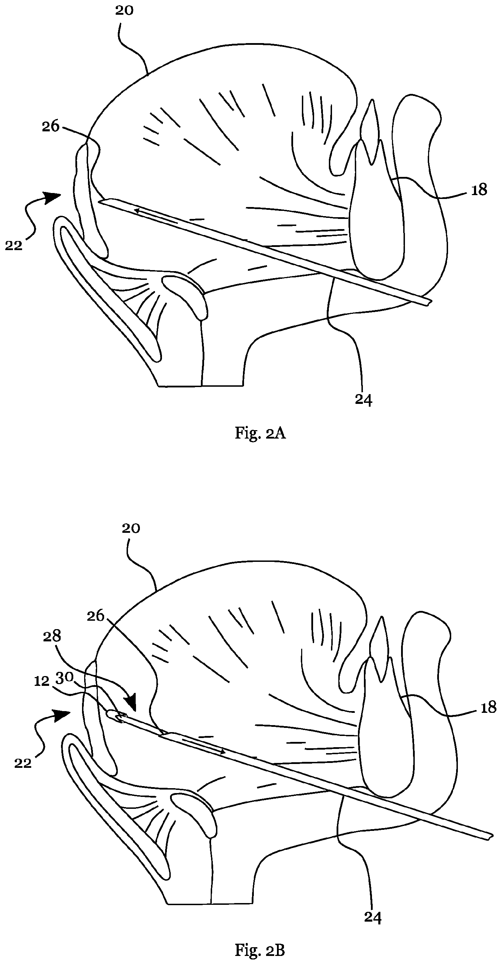

Fig. 2A-3A illustrate example steps of an implantation process of the implant 10 shown in Fig. 1 in the tongue of a patient based on a schematic sectional side view of the lower jaw.

As shown in the example of Fig. 2A, the implantation process may be commenced by introducing a cannula 24, such as a cannula 24 of a trocar for minimally invasive surgery into the lower jaw of the patient, wherein the cannula 24 is advanced towards a desired implantation location of the distal anchor 12 in the tongue 20 of the patient. The cannula 24 may comprise an angled tip at its distal end 26 for cutting soft tissue along the insertion direction, and/or may hold an insert with a cutting tip for advancing the cannula 24 towards the desired implantation location of the distal anchor 12 while radially deflecting the soft tissue along the insertion path.

As shown in the example of Fig. 2B, a balloon catheter 28 with a balloon 30 at its distal end 26 may be inserted through the cannula 24 towards the desired implantation location for the distal anchor 12. The balloon 30 may carry the distal anchor 12 in a collapsed state of the distal anchor 12, in which the distal anchor 12 may be crimped on the balloon 30 with a diameter of less than about 5 mm. The cannula 24 may subsequently be partially retracted, such that the balloon 30 carrying the distal anchor 12 may exit the cannula 24 at the distal end 26 of the cannula 24 at the desired implantation location.

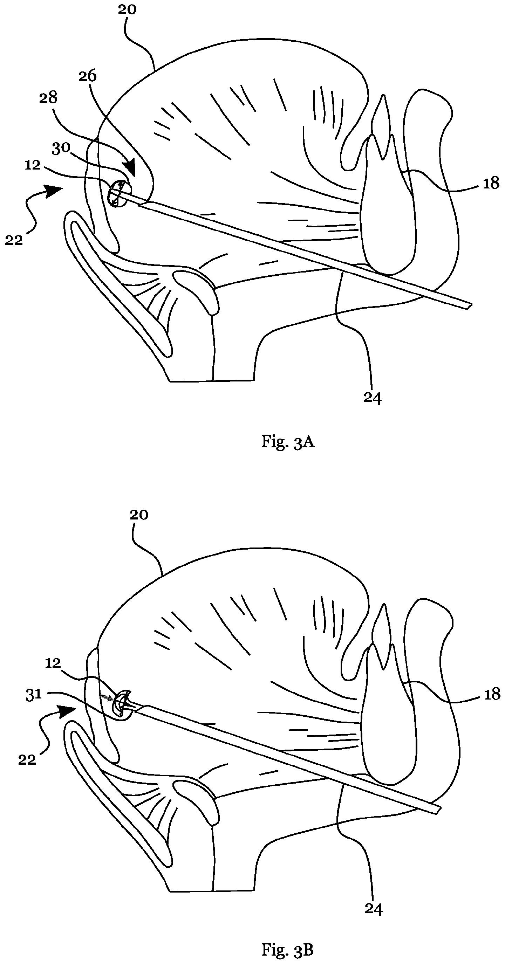

As shown in the example of Fig. 3A, the balloon 30 may be expanded from a collapsed state towards an expanded deployment state by inserting a fluid into the balloon catheter 28. In response to the influx of the fluid, such as pressurized gas, the balloon 30 may (e.g. radially) expand at the desired implantation location, such as to radially deflect surrounding soft tissue. The expansion of the balloon 30 may be uniform, such as to create a substantially circular cavity in the soft tissue of the tongue 20.

When the distal anchor 12 is crimped on the balloon 30, the uniform expansion may induce a pressure on an inner surface of the distal anchor 12, which may transfer the distal anchor 12 from the collapsed state to an expanded deployed state, as shown in Fig. 3A. For example, the distal anchor 12 may comprise folding portions along which the distal anchor 12 is folded to assume the collapsed state, and the distal anchor 12 may unfold according to the folding portions to assume the expanded deployed state.

As a result of the unfolding, the distal anchor 12 may have a larger cross section, when viewed along the insertion direction of the cannula 24, and may span a larger perimeter along the inner surface of the cavity induced by the balloon 30 in the soft tissue of the tongue, when the balloon is in the expanded deployment state.

While the balloon 30 is expanded towards the expanded deployment state, a surgeon may fix or apply a pulling force to a connection element 14 (not shown in Fig. 3A) to which the distal anchor 12 is attached and which is routed through the cannula 24 towards a proximal end of the cannula 24, such as to apply a pulling force on the distal anchor 12 along the extension direction of the cannula 24. The pulling force may maintain a contact of the distal anchor 12 and the balloon 30, such that the distal anchor 12 is prevented from disengaging from the balloon 30, when the balloon is expanded towards its expanded deployment state. In some embodiments, the balloon catheter 28 features a channel (not shown in Fig. 3A) for guiding the connection element 14 along an extension of the balloon catheter 28, such as to facilitate insertion of the distal anchor 12 and the connection element 14 into the soft tissue and/or to define a pulling force direction during the expansion of the balloon 30 for transferring the distal anchor 12 from the collapsed state to the expanded deployed state.

Thus, the expansion of the balloon 30 may deflect the surrounding soft tissue against a restoring force of the tissue and may inelastically transform portions of the distal anchor 12 to deploy the distal anchor 12 at the desired implantation location.

The balloon 30 may be deflated subsequent to the transfer of the distal anchor 12 towards the expanded deployed state at the desired implantation location. For example, a fluid pressure applied to the balloon catheter 28 may be reduced, such that the fluid exits the balloon 30 via a proximal end of the balloon catheter 28. The distal anchor 12 is configured to maintain the expanded deployed state against a restoring force of the surrounding soft tissue, such that the surrounding tissue may wrap around the distal anchor 12 as the balloon 30 collapses.

When the balloon 30 is in a collapsed state, the balloon catheter 28 may be retracted through the surgical opening in the soft tissue, e.g. through the cannula 24 or together with the cannula 24. The connection element 14 may be unsheathed from the cannular 24 and/or the balloon catheter 28, such that, when the cannula 24 is removed from the surgical opening, the connection element 14 connects the distal anchor 12 to a proximal portion of a surgical cavity in the patient's soft tissue, e.g. a surgical incision in the lower jaw of the patient. In the deployed state, a lateral extension of the distal anchor 12 is increased with respect to the collapsed state, and soft tissue may fit snugly and/or wrap around the distal anchor 12, when the surgical cavity collapses after the balloon 30 is deflated and/or the cannula 24 is removed from the soft tissue.

The connection element 14 may subsequently be connected at an end opposite the distal anchor 12 to a fixation 16 on the lower jaw bone 18, e.g. by winding the connection element 14 around a fixation plate (not shown) fixed to a bone screw attached to the lower jaw bone 18 of the patient.

Further, additional components (not shown in Fig. 3A) of the implant 10 may be connected to the connection element 14, such as connectors for adjusting a length and/or a biasing force of the connection element 14 in a continuous or stepwise manner after the connection element 14 has been connected to the fixation, elastic elements and/or switchable elements.

Fig. 3B schematically illustrates an explantation process of the distal anchor 12 with an explantation element 31 according to an example. The explantation element 31 comprises a distal lever section, which comprises two half-shell shaped levers in the illustrated example. When an explantation of the implant 10 is desired, the explantation element 31 may be compressed and introduced towards the implantation position/site of the distal anchor 12 through a cannula 24. The explantation element 31 or the cannula 24 may comprise an inner channel for receiving the connection element 14, such as to guide a distal tip of the explantation element 31 to the distal anchor 12 along the connection element 14.

At the implantation position/site, the distal tip of the explantation element 31 may protrude from the cannula 24, and the distal lever section may expand, e.g. by separation of the two half-shell shaped levers from each other, such as to abut against radially outward portions of the distal anchor 12. For example, the explantation element 31 may comprise a distal lever section made of a metal, e.g. nitinol, which may be (super-)elastically deformed for introduction into the cannula 24, and the distal lever section may expand towards an uncompressed state, when the distal lever section exits the cannula 24, based on a restoring force of the distal lever section.

Subsequently, a pulling force may be applied to the connection element 14, such as to force the distal anchor 12 into engagement with radially outward portions of the distal lever section of the explantation element 31 for collapsing the distal anchor 12. The distal lever section may provide a radially outward pivot point for facilitating the collapse of the distal anchor 12, e.g. by folding of bars or flaps of the distal anchor 12, and the collapsed distal anchor 12 may subsequently be removed from the tongue 20 of the patient through the cannula 24.

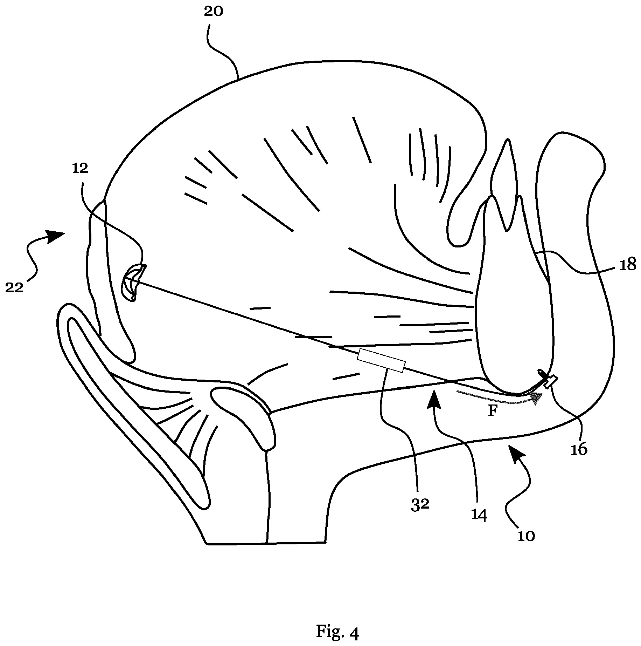

Fig. 4 illustrates a sectional side view of the patient's lower jaw with another example of an implant 10 in an implanted state similar to the example of Fig. 1. In the illustrated example, the connection element 14 comprises an elastic element 32 arranged between string-like sections of the connection element 14. The string-like sections may connect opposite sides of the elastic element 32 to a distal anchor 12 arranged at a desired implantation location in the tongue 20 of a patient and to a fixation 16 implemented with a bone screw attached to the lower jaw bone 18 of the patient, respectively.

The elastic element 32 may be introduced into the soft tissue of the tongue 20 during the retraction of the cannula 24 used for implantation of the distal anchor 12, or may be connected to a string-like section of the connection element 14 subsequent to the retraction of the cannula 24 from the soft tissue of the patient. The elastic element 32 may provide an adjustable or pre-determined elastic segment for the connection element 14, which may permit a relative distancing of the fixation 16 and the distal anchor 12 along the extension direction of the connection element 14 against a restoring force of the elastic element 32.

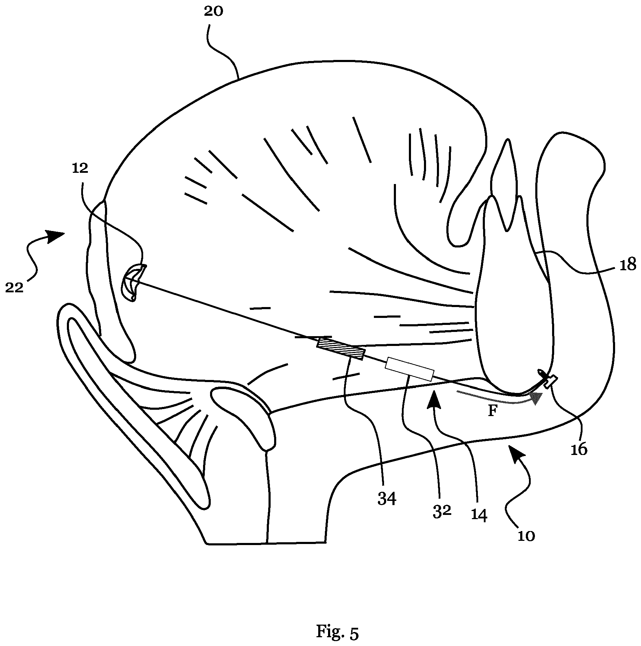

Fig. 5 illustrates a sectional side view of the patients lower jaw with yet another example of an implant 10 in an implanted state similar to the example of Fig. 4. In the illustrated example, the connection element 14 comprises an elastic element 32 arranged between string-like sections of the connection element 14, as well as a switchable element 34 configured to be selectively switched between a first state and a second state, wherein a mobility of the tongue 20 in the second state is increased. For example, the first state may have a shorter length along the extension direction of the connection element 14 than the second state, such that the connection element 14 is effectively lengthened, when the switchable element 34 is switched to the second state.

The switchable element 34 should be switchable from the first state to the second state, when the implant 10 is finally implanted in the soft tissue, wherein direct access to the switchable element 34 is prevented by the soft tissue surrounding the switchable element 34. Thus, the patient may switch the switchable element 34 from the first state to the second state, while the implant 10 is implanted, such as to selectively increase a mobility of the soft tissue, in which the distal anchor 12 is anchored. For example, the first state of the switchable element 34 may correspond to a state in which the tongue 20 is biased away from the back of the throat of the patient, e.g. during sleep, and the second state may be a state, which the patient may selectively activate for increasing tongue mobility, e.g. while not sleeping, such as during speech or eating.

The switchable element 34 may comprise a switch, which may be externally actuated by the patient, e.g. through exerting mechanical pressure on the soft tissue overlying the implant 10 on the lower jaw of the patient, such as to actuate a mechanical switch of the switchable element 34. Additionally or alternatively, the switchable element 34 may switch from the first state to the second state in response to a characteristic muscle contraction, such as a muscle contraction in conjunction with a certain tongue movement, e.g. the patient sticking out his tongue 20.

The switchable element 34 may revert to the first state after a pre-determined amount of elapsed time or may be switched back to the first step based on another actuation of the switch, or based on an actuation of another mechanical switch.

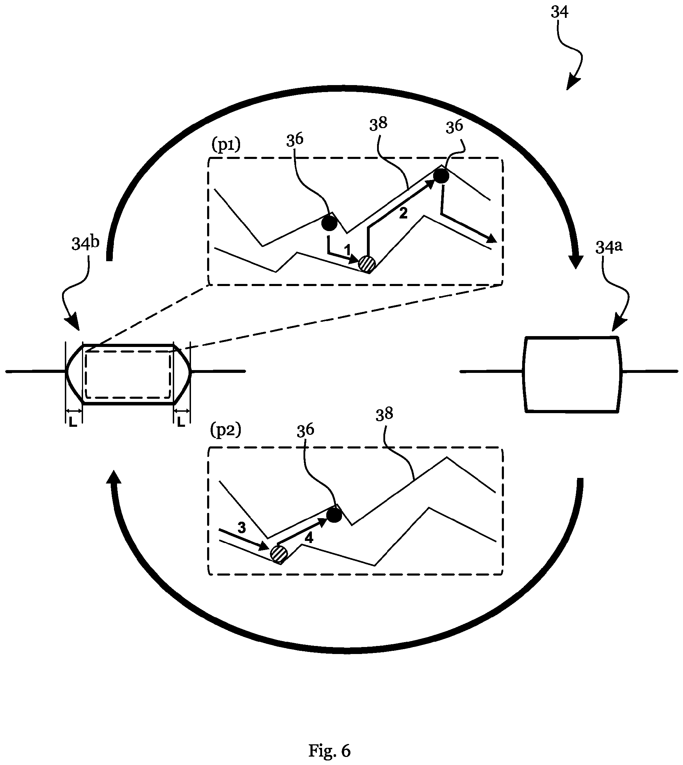

Fig. 6 illustrates a schematic example of the switching of a switchable element 34, 34a, 34b, which can be switched back and forth between a first state 34a, and a second state 34b. The solid arrows illustrate the switching from one state 34a, 34b to another state 34b, 34a, wherein the panels (p1), (p2) illustrate examples of a mechanical mechanism for providing the transfer between the states 34a, 34b.

The mechanism may operate in analogy to the mechanism of a retractable pen, wherein a pin 36 is housed in a housing 38 which may rotate around an axis and wherein the pin 36 is linear moveable along an axis orientation (vertical direction in Fig. 6) of said axis. The housing 38 comprises a jagged wall profile which constrains the movement of the pin 36 along the axis orientation, and the pin 36 is preloaded towards the top edge of the housing 38.

In an initial state, illustrated in panel (1), the pin 36 rests in a groove of the top wall of the housing 38. When the pin 36 is urged towards the bottom edge of the jagged wall profile, the housing 38 may turn along the arrow indicated with numeral "1" towards an intermediate state (dashed filling). If the force on the pin 36 is relaxed, the pin 36 can be urged according to the preload against the top edge of the wall profile of the housing 38, causing the state transfer along the arrow indicated with numeral "2". As a result of the shape of the housing 38, the position of the pin 36 along the axis direction is shifted, which may adjust a length of the switchable element 34. The length of the switchable element 34 may be according to a shift of the pin 36 along said axis direction by a length difference "L", as indicated for the second state 34b. Thus, the panel (p1) may indicate a state transfer from the second state 34b to the first state 34a.

When the pin 36 is again urged downwards, the jagged wall profile of the housing 38 may promote a combined pin motion and housing rotation along the arrow indicated with numeral "3", and when the force on the pin 36 is subsequently relaxed, the pin 36 can relax along the arrow indicated with numeral "4" to a configuration, which may correspond to the initial position of panel (p1). Thus, the panel (p2) may indicate a state transfer from the first state 34a to the second state 34b, and actuating a switch connected to the pin 36 may switch the switchable element 34 back and forth between a first state 34a and a second state 34b associated with different length dimensions, and differing by a length difference of 2"L" in the illustrated example.

The skilled person will appreciate that the illustrated switchable element 34 merely illustrates one possible implementation for a latch mechanism, which may switch a switchable element 34 between two states 34a, 34b of different length in response to the application of a mechanical force to the implant 10 in an implanted state, and that other implementations may be used in embodiments, such as other bi-stable mechanisms suitable for implementing a clicking pen, e.g. based on a bi-stable cam system.

In other examples, the switchable element 34 may be actively switched from the first state 34a to the second state 34b, e.g. based on an application of a mechanical force, but a return mechanism may be configured to return the switchable element 34 to the first state 34a after a pre-determined amount of elapsed time.

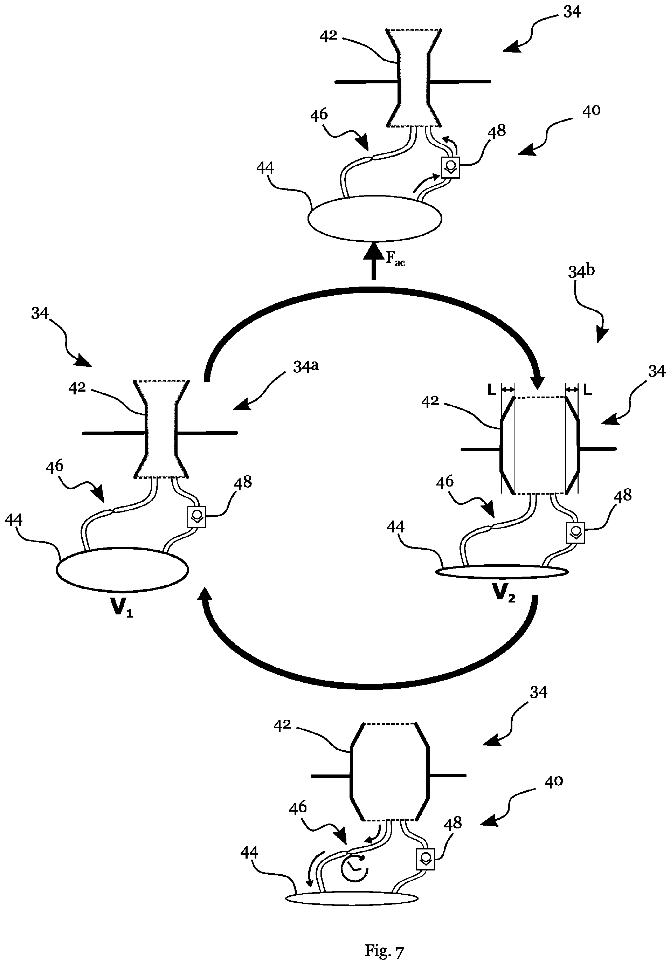

Fig. 7 schematically illustrates an example of a return mechanism 40 for a switchable element 34 suitable for an implant 10 as shown above. The return mechanism 40 comprises an expander vessel 42 and a storage vessel 44, wherein the expander vessel 42 and the storage vessel are connected via a first fluid path and a second fluid path, the first fluid path comprising a flow constriction element 46, and the second fluid path comprising a one-way fluid valve 48, wherein the first fluid path and the second fluid path are suitable for guiding a gel or liquid from the storage vessel 44 to the expander vessel 42 and vice-versa. In the example, the flow constriction element 46 comprises a tube section with a reduced cross-section, such that transfer of gel or liquid through the flow constriction element 46 is reduced, as compared to other sections of the fluid path(s). In this example the one-way fluid valve 48 is implemented as a ball valve, such that a gel or liquid may flow from the storage vessel 44 to the expander vessel 42, but not in the opposite direction. A flow resistance for a flow of a gel or liquid from the storage vessel 44 to the expander vessel 42 through the one-way fluid valve 48 should be lower than a flow resistance for a flow of a gel or liquid through the flow constriction element 46.

When the switchable element 34 is in the first state 34a, an actuating force F_{ac} may be applied to the storage vessel 44, thereby forcing the gel or liquid in the storage vessel 44 to flow towards the expander vessel 42 through the one-way fluid valve 48. A flow of the gel or liquid from the expander vessel 42 to the storage vessel 44 may be impeded by the flow constriction element 46, such that a volume of the gel or liquid of the storage vessel 44 may be transferred to the expander vessel 42. The expander vessel 42 may be coupled to a bistable element of the switchable element 34, such as a metallic element comprising pre-bent walls of a superelastic alloy, and the additional volume of the gel or liquid in the expander element 42 may switch a state of the bistable element, e.g. by snapping into a different configuration.

For example, the expander vessel 42 may be coupled to a metallic body with predetermined folding portions, wherein portions of the metallic body may snap back and forth between two configurations based on a volume of the gel or liquid in the expander vessel 42 to adjust an effective length of the switchable element 34.

The metallic body may be made of a superelastic alloy, in particular a nickel titanium alloy, and a metallic element may reversibly deform to a stressed state in a stress induced phase to transfer the switchable element 34 from the first state to the second state, by snapping into a stressed meta-stable configuration.

The different configurations of the metallic element may be associated with a different effective length of the connection element 14, such that the mobility of the soft tissue anchored by the implant 10 may be adjusted based on the state 34a, 34b of the switchable element 34. One of the configurations, e.g. related to the second state 34b may be meta-stable, such as to promote a switching of the switchable element 34 to the first state 34a.

However, since the flow through the flow constriction element 46 is limited, the metallic body may maintain the second state 34b for a pre-determined time period, wherein the pre-determined time may be based on a maximum flow rate of the flow constriction element 46, e.g. an elapsed time until a restoring force acting on the expander vessel 42 has forced a pre-determined volume of the gel or liquid through the flow constriction element 46.

The gel or liquid may be a viscous liquid, such as a polymer, e.g. a boron based silicone, and may further be a rheopectic and/or dilatant liquid whose viscosity is dependent on time and/or sheer stress, for example boron-organo-silicon oxide polymers. A mechanism comprising a rheopectic liquid as the gel or liquid may be resistant to rapid tissue movement, such as muscle contractions during speech or swallowing for a tongue implant 10.

In the illustrated example, a flow of the gel or liquid from the storage vessel 44 to the expander vessel 42 may be initiated by applying pressure to the storage vessel 44. However, the skilled person will appreciate that the flow may also be initiated based on a different force, e.g. a pulling force acting on the switchable element 34, e.g. along an extension of the connection element 14, which may be caused by a muscle contraction of the soft tissue.

The skilled person will further appreciate that a mechanical switching of the switchable element 34 is merely an example of implementation, and that other ways of switching the switchable element 34 from the first state 34a to the second state 34b may be used in embodiments. For example, the switchable element 34 may be switchable in response to a magnetic force exerted on the switchable element 34, or in response to a switching command, e.g. transmitted wirelessly to the switchable element 34 and executed by an actuator of the switchable element 34.

Moreover, although the invention has been illustrated in conjunction with a support of the human tongue 20 with the implant 10, the skilled person will appreciate that the implant 10 may be used in other applications, such as for supporting other tissue sections, which may or may not be related to a medical condition, e.g. in cosmetic surgery.

The description of the preferred embodiments and the figures merely serve to illustrate the invention and the beneficial effects associated therewith, but should not be understood to imply any limitation. The scope of the invention is to be determined solely by the appended claims.

### LIST OF REFERENCE SIGNS

- 10: implant
- 12: distal anchor
- 14: connection element
- 16: fixation
- 18: jaw bone
- 20: tongue
- 22: back surface
- 24: cannula
- 26: distal end
- 28: balloon catheter
- 30: balloon
- 31: explantation element
- 32: elastic element
- 34: switchable element
- 36: pin
- 38: housing
- 40: return mechanism
- 42: expander vessel
- 44: storage vessel
- 46: flow constriction element
- 48: one-way fluid valve

## Claims

1. An implant (10) for supporting soft tissue, the implant (10) comprising:
a distal anchor (12) for implantation in the soft tissue, the distal anchor (12) having a collapsed state and a deployed state, the deployed state having a larger lateral extension than the collapsed state;
a connection element (14) attached to the distal anchor (12) for supporting the soft tissue according to a biasing force applied along the connection element (14); and
an expandable element (30), wherein the expandable element (30) is configured to receive a fluid to expand the expandable element (30) to an expanded deployment state for transferring the distal anchor (12) from the collapsed state to the deployed state;
wherein the distal anchor (12) is configured to maintain the deployed state against a restoring force of the tissue of the patient when the expandable element (30) is collapsed from the expanded deployment state.

2. The implant (10) of claim 1, wherein the expandable element (30) comprises an expandable balloon of a balloon catheter (28).

3. The implant (10) of claim 2, wherein the distal anchor (12) is configured to rest on the balloon (30) during implantation of the implant (10).

4. The implant (10) of claim 2 or 3, wherein the balloon catheter (28) comprises a guiding slot for guiding the connection element (14) from a distal portion of the balloon catheter (28) to a proximal portion of the balloon catheter (28);
wherein the guiding slot is in particular configured to guide a holding force applied to the connection element (14) from the proximal portion of the balloon catheter (28) to the distal anchor (12), such that the connection element (14) can maintain a contact between the distal anchor (12) and the expandable element (30), while the expandable element (30) is expanded.

5. The implant (10) of any one of the preceding claims, wherein the distal anchor (12) is crimped onto the expandable element (30) in the collapsed state of the distal anchor (12) prior to deploying the implant (10).

6. The implant (10) of any one of the preceding claims, wherein the distal anchor (12) comprises a plurality of folding portions, wherein the distal anchor (12) is configured to fold along the folding portions to transfer the distal anchor (12) from the collapsed state to the deployed state.

7. The implant (10) of any one of the preceding claims, wherein the implant (10) further comprises a switchable element (34), wherein the switchable element (34) is configured to be switched from a first state (34a) to a second state (34b), wherein the first state (34a) is associated with a first length, wherein the second state (34b) is associated with a second length, and wherein the first length is different from, in particular smaller than, the second length.

8. The implant (10) of claim 7, wherein the switchable element (34) comprises a switch and actuating the switch transforms the switchable element (34) from the first state (34a) to the second state (34b);
wherein the switch is in particular configured to urge a latch mechanism to switch back and forth between two configurations, when the switch is actuated, such that the switchable element (34) switches from the first state (34a) to the second state (34b), and vice-versa.

9. The implant (10) of claim 7 or 8, wherein the switchable element (34) is configured to be switched from the first state (34a) to the second state (34b) based on a muscle contraction of the patient.

10. The implant (10) of any one of claims 7 to 9, wherein the switchable element (34) comprises a return mechanism (40), wherein the return mechanism (40) returns the switchable element (34) from the second state (34b) to the first state (34a) after a predetermined time period;
wherein the return mechanism (40) in particular comprises a viscoelastic element, wherein a gel or liquid flows from an expander vessel (42) to a storage vessel (44) to return the switchable element (34) from the second state (34b) to the first state (34a) after the predetermined time period.

11. The implant (10) of any one of claims 7 to 10, wherein the switchable element (34) comprises a switching element made of a superelastic alloy, in particular a nickel titanium alloy, and the switching element reversibly deforms to a stressed state in a stress induced phase to transfer the switchable element (34) from the first state (34a) to the second state (34b).

12. The implant (10) of any one of the preceding claims, wherein the implant (10) is for treating obstructive sleep apnea, and the distal anchor (12) is configured for implantation in a tongue (20) of a patient;
wherein the connection element (14) in particular comprises an elastic portion (32), wherein a biasing force of the elastic portion (32) is configured or configurable for allowing tongue (20) movement of the patient, but preventing a gravitational collapse of the patient's airway.

13. An implant kit comprising the implant (10) according claim 12, wherein the implant kit comprises a cannula (24) for introducing the distal anchor (12) on the expandable element (30) into the tongue (20) of the patient;
wherein the implant kit in particular further comprises a fixation (16) for fixedly attaching the implant (10) to a bone portion (18) of the patient, preferably to a lower jaw bone portion (18) of the patient, wherein, in a deployed state of the implant (10), the fixation (16) is connected to the distal anchor (12) through the connection element (14).

14. The implant kit of claim 13, further comprising a fixation plate configured for attachment of the connection element (14) to a bone portion (18) of the patient;
wherein the fixation plate is in particular configured to protrude towards the tongue (20) of the patient, when the implant (10) is deployed in the tongue (20) of a patient, such that the connection element (14) is prevented from directly contacting a bone portion (18) of the patient, when the distal anchor (12) is biased towards an attachment portion of the fixation plate by the connection element (14).

15. The implant kit of claim 13 or 14, wherein the lateral extension of the distal anchor (12) in the collapsed state and/or the inner diameter and/or the outer diameter of the cannula (24) is smaller than 6 mm.
